Europäisches Patentamt

(19) European Patent Office

Office européen des brevets

(11) Veröffentlichungsnummer: **0 035 583**
B1

(12) # EUROPÄISCHE PATENTSCHRIFT

(45) Veröffentlichungstag der Patentschrift:
14.08.85

(51) Int. Cl.⁴: **A 61 F 13/00**, A 61 M 35/00

(21) Anmeldenummer: **80101233.7**

(22) Anmeldetag: **11.03.80**

(54) **Hauttransplantations-Druckverband.**

(43) Veröffentlichungstag der Anmeldung:
**16.09.81 Patentblatt 81/37**

(45) Bekanntmachung des Hinweises auf die Patenterteilung:
**14.08.85 Patentblatt 85/33**

(84) Benannte Vertragsstaaten:
**AT BE CH FR GB IT NL SE**

(56) Entgegenhaltungen:
**DE - C - 449 589**
**DE - C - 848 234**
**DE - U - 7 806 873**
**FR - A - 1 255 501**
**US - A - 3 367 332**
**US - A - 4 202 331**

**F. SMITH; "Plastic and Reconstructive Surgery" 1950,**
**W.B. SAUNDERS CO., Philadelphia & London**

(73) Patentinhaber: **Schmid, Eduard, Dr.Dr.med.,**
**Böhelmstrasse 37, D-7000 Stuttgart 1 (DE)**

(72) Erfinder: **Schmid, Eduard, Dr.Dr.med.,**
**Böhelmstrasse 37, D-7000 Stuttgart 1 (DE)**

(74) Vertreter: **Patentanwälte Kohler - Schwindling - Späth,**
**Hohentwielstrasse 41, D-7000 Stuttgart 1 (DE)**

**Beschreibung**

Die Erfindung bezieht sich auf einen Hauttransplantations-Druckverband mit einem Kissen zum Andrücken von in Körpern eingesetzten Haupttransplantaten und mit Mitteln zum Erzeugen eines Gasüberdruckes im Kissen.

Ein derartiger Verband ist aus der Druckschrift von Ferris Smith: Plastic and Reconstructive Surgery, W.B. Saunders Co., 1950, Seiten 26 und 27, bekannt.

Bei der Transplantation von Haut, insbesondere dann, wenn die Haut in ihrer ganzen Dicke verpflanzt wird, ist es entscheidend, dass das ganze Transplantat bei einem gleichmässigen Druck auf die betreffende Körperstelle aufgedrückt wird. Dieser gleichmässige Druck muss sechs bis acht Tage aufrecht erhalten werden, er kann individuell etwas varriieren.

Es ist aus der genannten Druckschrift von Ferris Smith bekannt, zu diesem Zweck einen Druckverband anzulegen, der ein Kissen enthält, das grösser als das Hauttransplantat ist und dieses auf einer ganzen Fläche gleichmässig auf den wunden Körperteil aufdrückt.

Nachteile liegen jedoch darin, dass, da die bekannten Kissen aus Gummi bestehen, sich zwischen dem Kissen und der Oberfläche des Transplantates Eiweiss-Sekrete ansammeln und dass das Kissen verhindert, dass Luft an die Oberfläche des Transplantates gelangt. Schliesslich lässt sich auch bei Anwendung grösster Sorgfalt nicht immer vermeiden, dass die auf dem Transplantat aufliegende Fläche des Kissens durch den Druckverband Falten wirft, insbesondere wenn die Haut auf stark gekrümmte Körperpartien verpflanzt werden muss. Diese Falten bilden sich auf dem übertragenen Hautstück ab und sind auch nach dem völligen Abheilen sichtbar.

Aus der DE-U-7 806 873 ist noch eine Vorrichtung zum Ausspülen von Wunden bekannt, bei der eine flexible Hülle derart auf eine Wunde aufgeklebt wird, dass ein Kleberand um die Wunde herum verläuft und die Hülle die Wunde, ohne sie zu berühren, überdeckt. Durch einen Zu- und einen Abfluss kann ein Spülmittel zum Ausspülen der Wunde in die Hülle eingeleitet und aus dieser wieder abgesaugt werden.

Schliesslich ist aus der DE-C-449 589 noch eine Vorrichtung zum andauernden Einführen von Arzneimitteln o.dgl. durch die Haut von Menschen bekannt, die aus einem elastischen Häutchen aus Gummi o.dgl. besteht. Das Häutchen wird bläschenartig in Gestalt eines Pflasters auf die Haut aufgesetzt, und mittels einer Spritze wird ein Arzneimittel unter Überdruck unter das elastische Häutchen gespritzt. Diese bekannte Vorrichtung nach Art eines Pflasters ist jedoch nur für ausserordentlich kleine Bereiche vorgesehen, bei denen das Verhältnis von wirksamer Arzneimittel-Oberfläche zur Klebefläche relativ klein ist.

Der Erfindung liegt demgegenüber die Aufgabe zugrunde, einen Hauttransplantations-Druckverband der eingangs genannten Art dahingehend weiterzubilden, dass die Nachteile der bekannten Verbände vermieden werden. Insbesondere soll ein vollkommen gleichmässiger Druck über die gesamte Fläche des Hauttransplantates gewährleistet werden, ohne dass es zu einer Ansammlung von Eiweiss-Sekreten zwischen dem Verband und dem Hauttransplantat kommen kann.

Diese Aufgabe wird erfindungsgemäss dadurch gelöst, dass das Kissen an seiner dem Körper zugewandten Seite eine Aussparung aufweist, dass die Aussparung zumindest nahezu so gross ist wie das Transplantat, dass die Mittel als mindestens ein Ventil zum Anschluss einer Einrichtung zum Erzeugen, Aufrechterhalten und/oder Kontrollieren des Druckes innerhalb des Kissens ausgebildet sind und dass in das Kissen eine die Feuchtigkeit bindende Substanz eingelagert ist, wobei die die Feuchtigkeit bindende Substanz durch eine semi-permeable Wand von dem Hohlraum des Kissens getrennt ist, der mit dem Transplantat in Verbindung steht.

Beim Anlegen des Druckverbandes wird dann dieses Kissen so auf das Transplantat gelegt, dass die Aussparung über das Transplantat zu liegen kommt, und der Rand der Aussparung wird auf die Haut aufgeklebt.

Die Aussparung weist die Form des Hauttransplantates auf. Bei Ausführungsformen der Erfindung kann die Aussparung etwas kleiner sein als das Hauttransplantat, so dass der Rand der Aussparung noch auf dem Transplantat aufliegt, oder aber die Aussparung ist etwas grösser als das Transplantat, in welchem Falle der Rand der Aussparung auf der gesunden Haut aufliegt.

Das Transplantat kann in der üblichen Weise an den Rändern der Wunde angenäht sein, man kann jedoch auch auf die Naht verzichten, wenn bei der zuerst genannten Ausführungsform der Erfindung der Rand der Aussparung nicht nur auf den Rand des Hauttransplantates, sondern auch auf die gesunde Haut aufgeklebt wird.

Vorteile des erfindungsgemässen Kissens liegen zunächst einmal darin, dass im Bereich der Aussparung die Haut nicht schwitzt und die Absonderung von Eiweiss und anderen Sekreten entweder unterbleibt oder nicht zu einer Irritation der Haut führt. Die Massnahme, in dem Kissen eine Masse einzulagern, die Feuchtigkeit bindet und daher die im Kissen befindliche Luft trocknet, hat den Vorteil, dass sich in dem Kissen kein Schweiss oder Wundwasser ansammeln kann. Eine Rückwirkung von der Substanz auf das Transplantat ist dabei in vorteilhafter Weise dadurch ausgeschlossen, dass die semi-permeable Wand, die z.B. aus einem geeigneten Kunststoff bestehen kann, die Feuchtigkeit nur in Richtung zu der trocknenden Substanz hindurchlässt.

Durch die Aussparung hindurch wird das Transplantat belüftet. Ausserdem kann durch die Aussparung hindurch das Transplantat mit Sauerstoff oder aber einer Nährlösung versorgt werden, so dass hierdurch die Abheilung möglicherweise beschleunigt oder in den Abheilungsprozess korrigierend eingegriffen werden kann.

Schliesslich kann das Kissen im Bereich der Aussparung, also über dem Transplantat, keine Falten werden, so dass dieser Nachteil des bekannten Kissens vermieden wird.

Wenn die dem Körper abgewandte Seite des Kissens aus einem durchsichtigen Werkstoff besteht, so kann durch die Aussparung hindurch der Heilungsprozess kontrolliert werden bzw. der Grad der Durchblutung des Hauttransplantates beobachtet werden und, falls erforderlich, in der oben erwähnten Weise durch Füllen des Kissens mit einem Behandlungsmedium korrigiert werden. Daher besteht bei einer Ausführungsform der Erfindung das Kissen zumindest im Bereich der der Aussparung gegenüber liegenden Seite aus einem durchsichtigen Werkstoff, beispielsweise aus einer geeigneten Kunststoffolie o.dgl.

Die Aussparung im Kissen kann unmittelbar vor Anlegen des Druckverbandes in der Form des Hauttransplantates aus dem Kissen ausgeschnitten werden. Es können auch Kissen mit verschieden grossen, vorgefertigten Aussparungen hergestellt und dem Chirurgen zur Verfügung gehalten werden.

Bei einer Ausführungsform der Erfindung weist das Kissen auf seiner dem Körper abgewandten Seite eine steife Platte auf, die entsprechend der vorerwähnten Ausführungsform auch durchsichtig sein kann. Diese Platte kann der Körperform angepasst sein, auf die das Kissen aufgelegt werden soll.

Diese Ausführungsform der Erfindung hat den Vorteil, dass die Gefahr, dass sich beim Anlegen des Druckverbandes Falten in dem nicht ausgesparten Bereich der auf dem Körper aufliegenden Seite des Kissens bilden, wesentlich verringert ist.

Das erfindungsgemässe Kissen kann mit einer elastischen Binde mit oder ohne Zwischenschaltung der steifen Platte auf den Körper aufgedrückt werden, an den Extremitäten kann zu diesem Zweck auch ein elastischer Strumpf verwendet werden.

Bei einer Ausführungsform der Erfindung ist nur ein Ventil vorgesehen, das zum Erzeugen, zum Erhalten oder Kontrollieren des Druckes innerhalb des Kissens und/oder zum Einfüllen einer Flüssigkeit in das Innere des Kissens geeignet ist, oder aber es sind zwei Anschlüsse vorgesehen, so dass Luft und Flüssigkeit innerhalb des Kissens ausgetauscht werden können, ohne dass sich der Druck im Kissen wesentlich ändert.

Eine im Kissen befindliche Flüssigkeit kann durch Einleiten von Luft in den einen Anschluss zum anderen Anschluss hinausgedrückt werden und umgekehrt im Kissen befindliche Luft durch den einen Anschluss aus dem Kissen verdrängt werden. Die beiden Anschlüsse können auch dazu verwendet werden, um die im Kissen befindliche, unter Druck stehende Flüssigkeit oder unter Druck stehende Luft ständig zu erneuern oder durch ein Gerät zirkulieren zu lassen, das dieses Medium behandelt, beispielsweise der Luft Feuchtigkeit entzieht, so dass sich in dem Kissen kein Schweiss oder Wundwasser ansammelt. Selbstverständlich kann durch einen der Anschlüsse auch Flüssigkeit abgesaugt werden.

In der Zeichnung sind Ausführungsformen der Erfindung dargestellt, bei denen allerdings die die Feuchtigkeit bindende Substanz und die semipermeable Wand nicht eingezeichnet sind. Es zeigen:

Fig. 1 eine Ansicht auf diejenige Seite einer Ausführungsform des erfindungsgemässen Kissens, die zur Auflage auf der zu behandelnden Körperpartie bestimmt ist;

Fig. 2 einen Schnitt nach der Linie II–II durch die Ausführungsform nach Fig. 1;

Fig. 3 eine andere Ausführungsform der Erfindung in Seitenansicht;

Fig. 4 eine dritte Ausführungsform in einem der Fig. 2 entsprechenden Schnitt.

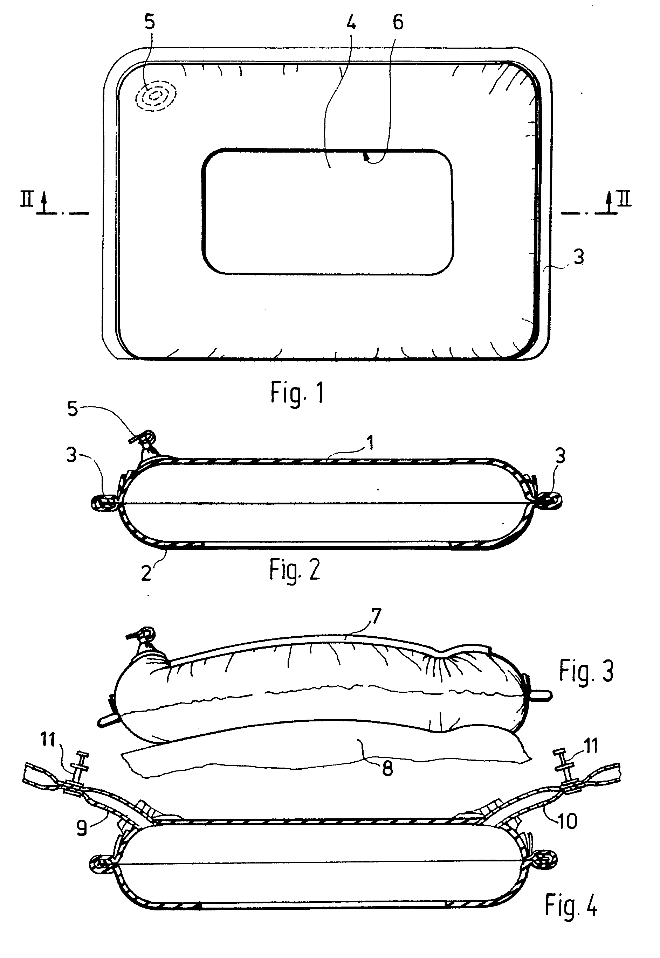

Bei der in Fig. 1 und 2 dargestellten Ausführungsform der Erfindung weist das Kissen zwei Folienzuschnitte 1 und 2 aus Latex oder einem geeigneten elastischen Kunststoff auf, die an ihren Rändern durch eine Naht 3 luftdicht miteinander verbunden sind. An derjenigen Seite des Kissens, die beim Anlegen des Druckverbandes auf die zu behandelnde Körperoberfläche aufgelegt wird, weist die Folie 2 eine Aussparung 4 auf. In der Nähe des Randes des Kissens ist in die Folie 1 ein Ventil 5 eingeschweisst. Die Aussparung 4 ist grösser als das Hauttransplantat, so dass der Rand 6 bei dieser Ausführungsform der Erfindung vollständig ausserhalb der Naht auf den Körper aufgeklebt wird, mit der das Hauttransplantat an der gesunden Haut befestigt wird. Das Ventil 5 ist so ausgebildet, dass an dieses bekannte Einrichtungen zum Erzeugen, Aufrechterhalten und Kontrollieren eines bestimmten Druckes innerhalb des Kissens angeschlossen werden können. Die Folie 1 ist durchsichtig, so dass durch diese Folie und durch die Aussparung 4 hindurch das Hauttransplantat beobachtet werden kann, ohne dass der Druckverband gelöst wird. Voraussetzung hierzu ist, dass in dem über dem Kissen angeordneten Teil des Verbandes ebenfalls ein Fenster vorgesehen wird, das diese Beobachtung zulässt, oder dass dieser Teil ebenfalls durchsichtig ist.

Die in Fig. 3 dargestellte Ausführungsform der Erfindung unterscheidet sich von der in den Fig 1 und 2 dargestellten Ausführungsform dadurch, dass auf die Folie 1 eine steife Platte 7 aufgeschweisst oder aufgeklebt oder auf andere Weise in ihrer Lage fixiert ist, die die Form der Oberfläche der zu behandelnden Körperpartie 8 aufweist. Diese Kunststoffplatte kann ebenfalls durchsichtig sein, um durch sie hindurch das Hauttransplantat beobachten zu können. Ihre Steifigkeit und ihre Grösse ist so gewählt, dass sie den von dem Druckverband ausgeübten Druck gleichmässig über die Oberfläche des Kissens verteilt und dadurch eine Faltenbildung in dem von der Folie 2 eingenommenen Bereich der Unterseite des Kissens verhindert.

Das Ventil 5 kann so ausgebildet sein, dass es auch zum Einführen eines Behandlungsmediums, gegebenenfalls unter Aufrechterhaltung des eingestellten Druckes im Kissen, geeignet ist. Auch kann für diesen Zweck ein zusätzliches Ventil am Kissen vorgesehen sein.

Fig. 4 zeigt eine Ausführungsform, die zwei Zuleitungen 9 und 10 aufweist, die in das Innere des Kissens münden. An diese Zuleitung können Geräte beliebiger Art angeschlossen werden, die das in dem Innern des Kissens befindliche Medium ohne Druckabfall austauschen, reinigen oder auf einem einstellbaren Druck halten. Zur Demonstration sind in Fig. 4 sehr einfache Absperrorgane 11 für diese Zuleitungen eingezeichnet, die bei der praktischen Ausführung der Erfindung seitlich durch geeignetere Geräte ersetzt sind.

Die Aussparung 4 kann abweichend von dem in Verbindung mit Fig. 1 beschriebenen Ausführungsbeispiel so gross sein, dass ihr Rand 6 teils auf der gesunden Haut, teils auf dem Transplantat, oder aber ganz auf dem Transplantat aufliegt.

Das Kissen kann mit einer Binde oder einem elastischen Strumpf auf das Transplantat aufgedrückt sein. Auch kann eine der beiden Folien 1 oder 2, insbesondere die untere Folie 2, wesentlich grösser als die andere Folie und über die Naht 3 hinaus verlängert sein und zum Befestigen des Kissens dienen.

## Patentansprüche

1. Hauttransplantations-Druckverband mit einem Kissen zum Andrücken von in Körpern (8) eingesetzten Hauttransplantaten und mit Mitteln zum Erzeugen eines Gasüberdruckes im Kissen, dadurch gekennzeichnet, dass das Kissen an seiner dem Körper (8) zugewandten Seite eine Aussparung (4) aufweist, dass die Aussparung (4) zumindest nahezu so gross ist wie das Transplantat, dass die Mittel als mindestens ein Ventil (5) zum Anschluss einer Einrichtung zum Erzeugen, Aufrechterhalten und/oder Kontrollieren des Druckes innerhalb des Kissens ausgebildet sind und dass in das Kissen eine die Feuchtigkeit bindende Substanz eingelagert ist, wobei die die Feuchtigkeit bindende Substanz durch eine semipermeable Wand von dem Hohlraum des Kissens getrennt ist, der mit dem Transplantat in Verbindung steht.

2. Verband nach Anspruch 1, dadurch gekennzeichnet, dass die Gestalt der Aussparung (4) so gewählt ist, dass ihr Rand (6) auf den Rand des Transplantates zu liegen kommt.

3. Verband nach Anspruch 1, dadurch gekennzeichnet, dass die Aussparung (4) grösser als das Transplantat gewählt ist und ihre Form so gewählt ist, dass der Rand (6) der Aussparung (4) zumindest teilweise auf der gesunden Haut aufliegt.

4. Verband nach einem der vorhergehenden Ansprüche, dadurch gekennzeichnet, dass das Kissen zumindest auf der der Aussparung (4) gegenüberliegenden Seite aus einem durchsichtigen Werkstoff besteht.

5. Verband nach einem der vorhergehenden Ansprüche, dadurch gekennzeichnet, dass das Kissen ein Ventil (5) aufweist, das zum Einführen eines Behandlungsmediums in den Raum innerhalb des Kissens geeignet ist.

6. Verband nach einem der vorhergehenden Ansprüche, dadurch gekennzeichnet, dass der Rand (6) der Aussparung (4) mit einem Klebstoff zum Befestigen des Kissens auf der Haut versehen ist.

7. Verband nach einem der vorhergehenden Ansprüche, dadurch gekennzeichnet, dass das Kissen auf der dem Körper (8) abgewandten Seite eine steife Platte (7) aufweist.

8. Verband nach Anspruch 7, dadurch gekennzeichnet, dass die Platte (7) der Form der Körperpartie (8) angepasst ist, zu deren Bedeckung das Kissen bestimmt ist.

9. Verband nach einem der vorhergehenden Ansprüche, dadurch gekennzeichnet, dass das Kissen zwei Anschlussstutzen (9, 10) aufweist.

10. Verband nach Anspruch 9, dadurch gekennzeichnet, dass die Anschlussstutzen ein Absperrorgan (11) aufweisen.

## Claims

1. Skin transplantation pressure dressing with a pad for pressing on to skin transplants placed in bodies (8) and with means for producing a gas overpressure in the pad, characterised in that the pad comprises a recess (4) on its side facing the body (8), the recess (4) is at least almost as big as the transplant, the means are in the form of at least one valve (5) for the connection of a device for generating, maintaining and/or checking the pressure inside the pad, and a moisture-binding substance is incorporated in the pad, the moisture-binding substance being separated by a semi-permeable wall from the cavity of the pad which is in contact with the transplant.

2. Dressing as claimed in claim 1, characterised in that the configuration of the recess (4) is selected so that its edge (6) comes to lie against the edge of the transplant.

3. Dressing as claimed in claim 1, characterised in that the recess (4) is made larger than the transplant and its shape is selected so that the edge (6) of the recess (4) abuts at least partially on the healthy skin.

4. Dressing as claimed in one of the preceding claims, characterised in that the pad consists of a transparent material, at least on the side opposite the recess (4).

5. Dressing as claimed in one of the preceding claims, characterised in that the pad has a valve (5) which is adapted for introducing a treatment medium into the space within the pad.

6. Dressing as claimed in one of the preceding claims, characterised in that the edge (6) of the recess (4) is provided with an adhesive substance for securing the pad to the skin.

7. Dressing as claimed in one of the preceding claims, characterised in that the pad has a rigid plate (7) on the side remote from the body (8).

8. Dressing as claimed in claim 7, characterised in that the plate (7) is adapted to the shape of the part of the body (8) which the pad is intended to cover.

9. Dressing as claimed in one of the preceding claims, characterised in that the pad has two connecting pieces (9, 10).

10. Dressing as claimed in claim 9, characterised in that the connecting pieces have a locking member (11).

**Revendications**

1. Bandage compressif pour transplantations de peau, comportant un coussin destiné à presser des transplants de peau insérés dans un corps (8) et des moyens destinés à produire une surpression gazeuse à l'intérieur du coussin, caractérisé en ce que le coussin présente, sur sa face adjacente au corps (8), un évidement (4), que l'évidement (4) est au moins aussi important que la transplant, que les moyens sont formés d'au moins une soupape (5) destinée au branchement d'un dispositif pour produire, maintenir et/ou contrôler la pression à l'intérieur du coussin et qu'il est inséré dans le coussin une substance ayant de l'affinité pour l'humidité, cette substance étant séparée par une paroi semi-perméable de la cavité du coussin qui est en communication avec le transplant.

2. Bandage selon la revendication 1, caractérisé en ce que la configuration de l'évidement (4) est choisie de telle sorte que son bord (6) vienne se placer sur le bord du transplant.

3. Bandage selon la revendication 1, caractérisé en ce que l'évidement (4) est plus important que le transplant et sa forme est choisie de telle sorte que le bord (6) de l'évidement (4) repose au moins en partie sur la peau saine.

4. Bandage selon l'une quelconque des revendications précédentes, caractérisé en ce que le coussin, au moins sur la face en regard de l'évidement (4), est réalisé en matière transparente.

5. Bandage selon l'une quelconque des revendications précédentes, caractérisé en ce que le coussin comporte une soupape (5) prévue pour introduire dans l'espace intérieur dudit coussin un milieu de traitement.

6. Bandage selon l'une quelconque des revendications précédentes, caractérisé en ce que le bord (6) de l'évidement (4) est muni d'un adhésif pour fixer le coussin à la peau.

7. Bandage selon l'une quelconque des revendications précédentes, caractérisé en ce que le coussin présente une plaque rigide (7) sur la face opposée au corps (8).

8. Bandage selon la revendication 7, caractérisé en ce que la forme de la plaque (7) est adaptée à celle de la partie du corps (8) que le coussin est destiné à recouvrir.

9. Bandage selon l'une quelconque des revendications précédentes, caractérisé en ce que le coussin présente deux ajutages (9, 10).

10. Bandage selon la revendication 9, caractérisé en ce que les ajutages comportent un organe de fermeture (11).

Fig. 1

Fig. 2

Fig. 3

Fig. 4